Europäisches Patentamt

European Patent Office

Office européen des brevets

(11) Publication number: **0 246 013**
A2

(12) **EUROPEAN PATENT APPLICATION**

(21) Application number: 87303953.1

(22) Date of filing: 01.05.87

(51) Int. Cl.4: **C08J 5/18** , C08L 89/06 , A61L 27/00

(30) Priority: 14.05.86 JP 110057/86

(43) Date of publication of application:
19.11.87 Bulletin 87/47

(84) Designated Contracting States:
BE CH DE FR GB IT LI NL SE

(71) Applicant: KOKEN CO. LTD.
5-18 Shimoochiai 3-chome
Shinjuku-ku Tokyo(JP)

(72) Inventor: Yoshizato, Katsutoshi
40-1-518, Oya
Ebina-shi Kanagawa-ken(JP)
Inventor: Taira, Toshio
2-28-26, Nakatehara Kohoku-ku
Yokohama-shi Kanagawa-ken(JP)
Inventor: Miyata, Teruo
3-6-29, Shimoochiai Shinjuku-ku
Tokyo(JP)

(74) Representative: Geering, Keith Edwin et al
REDDIE & GROSE 16 Theobalds Road
London WC1X 8PL(GB)

(54) **Method of making collagen film.**

(57) A method of producing a transparent collagen film having a high permeability to materials and suitable for culturing animal cells, comprises the steps of: forming a collagen film from an aqueous acid solution containing a collagen; dipping the collagen sheet in a saline solution; and radiating ultraviolet rays onto the collagen sheet.

EP 0 246 013 A2

# METHOD OF MAKING COLLAGEN FILM

The present invention relates to a method of producing a collagen film, preferably for use as a culture medium for culturing animal cells.

Conventionally, when animal cells are cultured on a culture medium, a cellulose-based membrane filter is often used as a culture medium. However, since this membrane filter is opaque, microscopic observations through the filter are impossible. Furthermore, when, for example, epidermal cells are cultured on a substrate and transplanted onto a living body as an artificial skin, the above cellulose-based membrane filter cannot be used as the culture medium because it lacks biocompatibility.

Various phenomena in the living body are caused by interactions between various types of cells. In order to study these phenomena, cells are isolated through a film and cultured. Therefore, a transparent film having high permeability to materials is desired.

In the method according to the present invention for forming collagen film, collagen sheet (suitably formed from aqueous acid containing collagen) is contacted with saline solution (e.g. by dipping) to form collagen fibers, and subjected to ultraviolet irradation to introduce crosslinks between the collagen fibers.

The method of the present invention can provide a transparent collagen film which has high permeability to materials and which is suitable for use in culturing animal cells.

The collagen used in the present invention may be atelocollagen obtained by treating acid soluble collagen or natural collagen with protease (such as pepsin) in aqueous acid solution (such as aqueous acetic) to remove telopeptides at terminal ends of the collagen molecule, and at the same time to cleave molecular crosslinks.

The acid used in the acid solution containing the collagen of the present invention may be hydrochloric acid.

The saline solution used in the present invention may be, for example, a phosphoric acid buffer saline solution of pH 5 to 9.

Epidermal cells can be effectively cultured on collagen film obtained by the present invention. This collagen film with the epidermal cells can be used for transplantation as an artificial skin.

Using collagen film produced by the present invention two types of cell can be separately cultured on the two surfaces of the film, thus allowing study of interactions between the two types of cell. When cells are cultured on one surface of the collagen film and a material such as elastin is coated on the other surface, chemotaxis of the cells towards the coated material can be studied.

The present invention will be more clearly understood from the following example; however, the example is intended to illustrate the invention and is not to be construed to limit the scope of the invention.

Example

An acidic solution containing 0.3 to 1.0% of bovine dermis atelocollagen prepared in a germ-free manner was poured into a mold frame placed in a hydrophobic container such that the solution level was 1 to 5 mm, and was air-dried to form an atelocollagen sheet. The sheet was removed from the mold frame. A circular sheet with a diameter of 33 mm was cut from the atelocollagen sheet and fitted in a plastic cylindrical frame to vertically divide the space in the frame. Subsequently, the cylindrical frame was dipped in a phosphoric acid-buffered saline solution (NaCl 150 mM, $Na_2HPO_4$ 0.02 M, pH 7.4) together with the circular sheet and was allowed to stand at 37°C for 1 hour to form collagen fibers. Ultraviolet irradiation (power: 10 W) was performed at a distance of 20 cm for 30 minutes to introduce crosslinks between the collagen fibers, thereby obtaining a transparent collagen film.

For the purpose of comparison, another atelocollagen sheet was prepared by air-drying by the same procedure as described above. This sheet was not dipped in a saline solution, but subjected to ultraviolet irradiation in the same manner as above in order to introduce crosslinks, thereby preparing a collagen film as a control.

Subsequently, an experiment was performed in the following manner in order to study the permeability of these two collagen films produced by the two different methods.

Two containers of an appropriate size each having two openings were prepared. The interior of each container was divided into two subchambers by a respective collagen film. Seven milliliters of a solution containing various proteins indicated in the follwing table were poured in one subchamber, and 7 mℓ of a solution without proteins were poured in the other subchamber. The container was maintained at 37°C for two days.

## Table

| | | | Molecular Weight |
|---|---|---|---|
| (1) | bovine serum albumin | | 66,000 |
| (2) | Ovalbumin | | 45,000 |
| (3) | Myoglobin | | 17,000 |
| (4) | $\alpha$-chymotrypsinogen | | 25,000 |
| (5) | Cytochrome | | 12,400 |
| (6) | Aprotinin | | 6,000 |

The collagen film which was not dipped in the saline solution did not have any permeability to these proteins.

On the other hand, the collagen film which was obtained by the method of the present invention has permeability to all of these proteins.

The measurements of permeability to these proteins were performed by SDS polyacrylamide electrophoresis.

The above experiment demonstrates that the collagen film of the present invention has a better permeability to materials.

Epidermal cells were cultured using the collagen films of this example and the control.

A collagen film obtained by the method of this example was fitted in a plastic cylindrical frame to vertically divide the interior of the frame and was placed in a laboratory dish containing a liquid culture medium. As the liquid culture medium, a Green's medium mainly consisting of an Eagle minimum essential medium (modified by Dulbecco) containing 10% fetal bovine serum and an antibiotic, and HAM F-12 at a ratio of 3 : 1 was used. In this case, the collagen film was dipped in the liquid culture medium such that no bubble is formed below the film. The distances between the collagen film and two ends of the cylindrical frame were set to 2 to 3 mm. Spacers having a height of 1 mm were placed under the lower end of the cylindrical frame so that the liquid culture medium portion under the collagen film and that outside the cylindrical frame could be freely substituted with each other.

Subsequently, about $1 \times 10^5$ epidermal cells obtained from human skin were inoculated only on the upper surface of the collagen film. The laboratory dish was placed in an incubator with a $CO_2$ concentration of 5% and at a temperature of 37°C for culturing the epidermal cells. Twenty days after the start of culturing, the epidermal cells proliferated by fission and spreaded over the entire surface of the collagen film.

On the other hand, using the collagen film of the control which was not dipped in a saline solution, the same culturing as above was performed. Twenty days after the start of culturing, the epidermal cells proliferated only at a part of the film.

## Claims

1. A method of producing collagen film comprising contacting collagen sheet with saline solution and subjecting the sheet to ultraviolet irradiation.

2. A method according to claim 1 wherein the saline solution is a phosphoric acid-buffered saline solution of pH 5 to 9.

3. A method according to claim 1 or 2 wherein the collagen is acid soluble collagen or atelocollagen.

4. A method according to any of claims 1 to 3 wherein the collagen sheet is formed from aqueous acid containing collagen.

5. A method according to claim 4 wherein the aqueous acid is an aqueous solution of hydrochloric acid.

6. A method of culturing cells in which a film made according to any preceding claim is employed as culture medium.